# EUROPEAN PATENT SPECIFICATION

(11) **EP 2 231 099 B1**
(45) Date of publication and mention of the grant of the patent: **24.04.2013**
(21) Application number: 08867115.1
(22) Date of filing: 22.12.2008
(51) Int. Cl.: A61J 1/20, A61J 1/10

(54) **MULTI-CHAMBERED CONTAINER**
MEHRKAMMERBEHÄLTER
RÉCIPIENT À PLUSIEURS COMPARTIMENTS

(30) Priority: 27.12.2007 US 17094 P
(43) Date of publication of application: 29.09.2010
(73) Proprietor: Baxter International Inc., Deerfield, IL 60015 (US); Baxter Healthcare S.A., 8152 Glattpark (Opfikon) (CH)
(72) Inventor: BALTEAU, Patrick, B-5350 Evelette (BE)
(74) Representative: Dee, Ian Mark
(86) International application number: PCT/US2008/088012
(87) International publication number: WO 2009/086300

(56) References cited:
- EP-A- 0 790 051
- EP-A- 1 621 177
- US-A1- 2002 166 779

## Description

### BACKGROUND

The present disclosure is directed to packaging products. More specifically, the present disclosure is directed to multi-chambered containers for the selective admixture of solutions, see for instance EP 0 790 051 A2.

Containers having sub-chambers are widely used to separately store two or more components. The components can be mixed inside the container and administered to a patient through a tube attached to the container. The components can be in a powder or liquid form and are typically mixed together to form a therapeutic solution. Such solutions can include intravenous solutions, nutritional solutions, drug solutions, enteral solutions, parenteral solutions, dialysis solutions, pharmacological agents including gene therapy and chemotherapy agents, and many other liquids that may be administered to a patient.

The storage chambers of the multi-chambered containers are often separated by a frangible heat seal. Peelable seals are among the frangible seals used that permit the seal to be separated by pulling on opposite sides of the container to mix the contents of the separated chambers. Nevertheless, multi-chambered containers are currently designed so that once the peel seal between chambers is broken, the entire contents from each chamber mixes together. As a result, these multi-chambered containers provide little control over the selective mixture of components in the storage chambers.

### SUMMARY

The present disclosure is directed to multi-chambered containers that can be used for the selective admixture of components within the chambers. In a general embodiment, the present disclosure provides a multi-chambered container including a first chamber, a second chamber, and a third chamber. A first peelable seal separates the first and third chambers and is independently openable by selective application of external pressure to the first chamber. A second peelable seal separates the second and third chambers and is independently openable by selective application of external pressure to the second chamber. The first chamber is separated from the second chamber by a permanent seal defining a transverse opening. At least one of the first chamber and second chamber is dimensioned to facilitate one-handed gripping of the chamber to apply the external pressure. The transverse opening is dimensioned to admit a human hand to apply the external pressure.

In an embodiment, the third chamber can contain a parenterally administrable base solution. In another embodiment, the third chamber can be empty and sized to contain the entire contents of the first chamber and the second chamber.

In an embodiment, the container is made from a film including one more materials such as, for example, polyolefins, polyamides, polyesters, polybutadiene, styrene and hydrocarbon copolymers, polyimides, polyester-polyethers, polyamide-polyethers, or a combination thereof. More specifically, the film can include one or more materials such as, for example, polyethylene homopolymers, ethylene α-olefin copolymers, polyethylene copolymers, polypropylene homopolymers, polypropylene copolymers, styrene and hydrocarbon random copolymers, styrene and hydrocarbon block copolymers, or a combination thereof.

In an embodiment, the first peelable seal and the second peelable seal are activated by a force ranging from about 3 N/15 mm to about 30 N/15 mm. In addition, the first peelable seal can have a first activating force and the second peelable seal can have a second activating force. The second peelable seal activating force can be greater than the first peelable seal activating force. The difference between the first peelable seal activating force and the second peelable seal activating force can be greater than about 1 N/15 mm and less than about 5 N/15 mm.

In an embodiment, at least a portion of the first peelable seal and the second peelable seal has a shape such as semicircular, rectangular, trapezoidal, polygonal, or a combination thereof.

In an embodiment, the first chamber and the second chamber contain components of a peritoneal dialysis solution. Alternatively, the first chamber and the second chamber can contain components of a parenteral nutrition solution.

In an embodiment, the multi-chambered container further includes a fluid outlet port in fluid communication with the third chamber. A peelable safety seal can separate the outlet port from the third chamber. The safety seal can have a seal strength selected to impede opening of the safety seal unless at least one of the first and second peelable seals have first been opened. The multi-chambered container can further include a tube in fluid communication with at least one of the first chamber and the second chamber.

In another embodiment, the multi-chambered container includes at least two plies of a flexible polymer film that define the first chamber, the second chamber, and the third chamber formed between the plies.

In an alternative embodiment, the present disclosure provides a multi-chambered container including at least two plies of a flexible polymer film defining a first chamber, a second chamber, a third chamber, and a fourth chamber formed between the plies. A first peelable seal separates the first and fourth chambers and is independently openable by selective application of external pressure to the first chamber. A second peelable seal separates the second and fourth chambers and is independently openable by selective application of external pressure to the second chamber. A third peelable seal separates the third and fourth chambers and is independently openable by selective application of external pressure to the third chamber. The first chamber is separated from the second chamber and the second chamber is separated from the third chamber by a permanent seal defining a transverse opening through the plies. At least one of the first chamber, second chamber, and the third chamber is dimensioned to facilitate one-handed gripping of the chamber to apply the external pressure. The transverse opening is dimensioned to admit a human hand to apply the external pressure.

In an embodiment, the fourth chamber is sized to facilitate mixing of the contents of any combination of the first, second, and third chambers. The multi-chambered container can further include a fluid outlet port in fluid communication with the fourth chamber. A peelable safety seal can separate the outlet port from the fourth chamber. The safety seal can have a seal strength selected to impede opening of the safety seal unless at least one of the first, second, and third peelable seals have first been opened. The container can also include one or more tubes in fluid communication with at least one of the first chamber, the second chamber, and the third chamber.

An advantage of the present disclosure is to provide an improved multi-chambered container have the capability for selectively administering the individual contents from specific chambers of the container.

Another advantage of the present disclosure is to provide an improved multi-chambered container that allows the reconstitution of custom tailored solutions.

Additional features and advantages are described herein, and will be apparent from the following Detailed Description and the figures.

### BRIEF DESCRIPTION OF THE FIGURES

FIG. 1 illustrates a front view of the multi-chambered container including two chambers that lead into a mixing chamber in an embodiment of the present disclosure.

FIG. 2 illustrates a front view of the multi-chambered container including three chambers that lead into a mixing chamber in another embodiment of the present disclosure.

### DETAILED DESCRIPTION

The present disclosure relates to multi-chambered containers having individually arranged storage chambers. In an embodiment, the multi-chambered containers allow an operator/user to selectively mix a specific amount of one or more components (e.g. solutions/concentrates) from each storage chamber into a holding/mixing chamber situated below the storage chambers containing the components. The holding/mixing chamber could be either empty at start of the mixing or be filled with a base solution that could potentially be infused as is (alone) to a patient. The mixing of the components container in the storage chambers can be performed by sequentially applying a specific amount of pressure on each of the individual storage chambers containing the specific components.

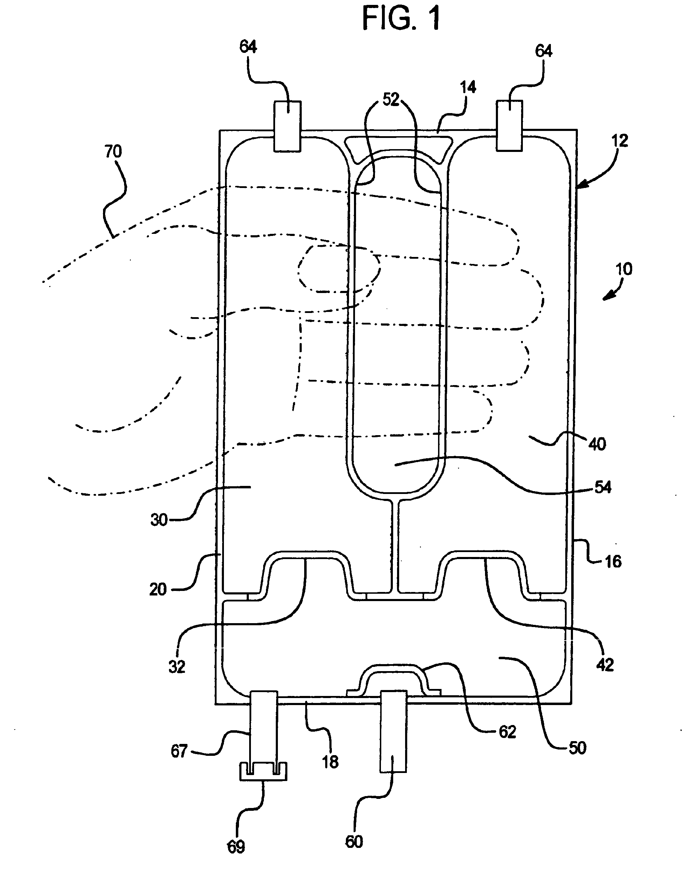

In a general embodiment shown in FIG. 1, the present disclosure provides a container 10 including a body 12 defined by a film. The container 10 includes a first chamber 30, a second chamber 40, and a third chamber 50. The container 10 may be made, for example, from two plies of the film that are sealed about the periphery of the container 10 at edges 14, 16, 18, and 20 to form outer permanent (cohesive) seals. In the illustrated embodiment, two plies of film (one for each side) are used although additional plies of film can be used. The size and dimensions of the container 10 and chambers 30,40, and 50 can vary depending on the application.

A first peelable (adhesive) seal 32 separates the first chamber 30 and the third chamber 50. The first peelable seal 32 is independently openable by selective application of external pressure to the first chamber 30. A second peelable seal 42 separates the second chamber 40 and the third chamber 50. The second peelable seal 42 is independently openable by selective application of external pressure to the second chamber 40. Accordingly, the component stored in the first chamber 30 can be added to chamber 50 separately from the component stored in the second chamber 40 and vice versa. Moreover, the amount of pressure separately applied to the first and second chambers 30 and 40 can control how much component is removed from the chambers.

The first chamber 30 is separated from the second chamber 40 by a permanent seal 52 defining a transverse opening 54. As a result, the first chamber 30 and/or the second chamber 40 can be dimensioned to facilitate one-handed gripping of the chambers 30 and 40 to apply the external pressure. For example, the transverse opening 54 positioned between the individual chambers 30 and 40 allows for a pressure to be applied on any one of the individual chambers 30 or 40 without exerting pressure on the other chambers.

By pressurizing, one at a time, a specific chamber (30 or 40) containing one or more components to be mixed together to satisfy the specific needs of a given patient, a user or care giver can prepare a mixed formulation having specified or selective amounts from the individually stored components of chambers 30 and 40. The chambers 30 and 40 can be marked with unit markings (e.g. showing volume remaining) to inform the user how much of the component has been removed from that chamber. The selective amount of the stored components can be thoroughly mixed in the third chamber 50 prior to being infused or administered to the patient.

The first peelable seal 32 and the second peelable seal 42 can be constructed and arranged to allow a precise amount of component to pass through between chambers. For example, a specified portion of the peelable seals 32 and 42 can open that corresponds to the amount of pressure or force applied to the chambers 30 and 40, respectively. In addition, the first peelable seal 32 and the second peelable seal 42 can be in the form of re-peelable/resealable seals that can be closed after a specified amount of component has been squeezed from their respective chambers into the third chamber 50.

In an embodiment, the transverse opening 54 has a width (side to side) greater than 5 mm. The transverse opening 54 can also have a larger width such as, for example, a width greater than 6 mm, greater than 7 mm, greater than 8 mm, greater than 9 mm, greater than 10 mm, and the like. In another embodiment, the transverse opening 54 has a length (top to bottom) greater than 5 cm. The transverse opening 54 can also have a larger length such as, for example, a width greater than 6 cm, greater than 7 cm, greater than 8 cm, greater than 9 cm, greater than 10 cm, greater than 11 cm, greater than 12 cm, greater than 13 cm, greater than 14 cm, greater than 15 cm, greater than 16 cm, greater than 17 cm, greater than 18 cm, greater than 19 cm, greater than 20 cm, and the like. The transverse opening 54 is dimensioned to admit a human hand 70 (e.g. adult/care giver) to apply the external pressure.

The first chamber 30 and the second chamber 40 can be storage chambers for holding one or more components such as a solution or concentrate. The third chamber 50 can be a holding/mixing chamber. For example, the components or solutions stored in chambers 30 and 40 can provide a reconstituted therapeutic solution when mixed together in chamber 50 prior to usage. Alternatively, the third chamber 50 can be pre-filled with a base solution that could be infused alone or in combination with one or more of the solutions/concentrates contained in the first chamber 30 and/or the second chamber 40, which can be selectively put in fluid communication with the third chamber 50. Such solutions can include intravenous solutions, nutritional solutions, drug solutions, enteral solutions, parenteral solutions, dialysis solutions, and many other liquids that may be administered to a patient.

In an embodiment, the first peelable seal 32 and the second peelable seal 42 can be activated by a force ranging from about 3 N/15 mm to about 30 N/15 mm. The force can be applied, for example, from a user squeezing sides of the container 10 so that the components stored within chambers 30 or 40 press against the first peelable seal 32 and the second peelable seal 42, respectively.

In another embodiment, the first peelable seal 32 has a first activating force (e.g. amount of force necessary to open the seal) and the second peelable seal 42 has a second activating force. The second peelable seal activating force is greater than the first peelable seal activating force. The difference between the first peelable seal activating force and the second peelable seal activating force can be greater than about 1 N/15 mm and less than about 5 N/15 mm.

The peelable seals 32 and 42 can have any suitable shape. For example, as illustrated in FIG. 1, the peelable seals 32 and 42 can be in the form of semi-circular seals having an apex that is oriented towards the inside of the individual chambers 30 and 40, respectively. In an embodiment, at least a portion of the peelable seals 32 and 42 has a shape such as semicircular, rectangular, trapezoidal, polygonal, or combinations thereof. The peelable seals 32 and 42 can also be serrated to facilitate their opening upon application of a pressure on the corresponding chamber.

As illustrated in FIG. 1, the container 10 can further include an administration tube 60 and a medication tube 67 in fluid communication with the third chamber 50. Medication tube 67 provides communication with the interior of chamber 50 and is equipped with a seal such as septum 69 that allows components such as a liquid to be added to or removed from the multi-chambered container, for example, after the contents of chambers 30 and 40 have been mixed. The tube 60 can also include a membrane (not shown) that seals shut the tube 60 and can be pierced by, for example, a cannula or spike of an administration set. The tube 60 can be sealed until the time to access the contents of the container 10.

A peelable safety seal 62 can surround an opening of the tube 60. The safety seal 62 can have a seal strength selected to impede opening of the safety seal 62 unless at least one of the first and second peelable seals 32 and 42 have first been opened. The peelable safety seal 62 can have any suitable shape.

in an embodiment, the container 10 can further include one or more tubes 64 in fluid communication with the first chamber 30 and/or the second chamber 40. The tubes 64 can provide communication with the interior of chambers 30 and 40 and allow components such as solutions/concentrates to be added to or removed from chambers 30 and 40. For example, the tubes 64 can be used as fill ports for chambers 30 and 40 or as additive ports to allow addition of a medication or other additive to one of the chambers after the chamber has been filled and sealed. The tubes 64 can be capped or sealed after the chambers 30 and 40 have been filled with the desired components. The number, size, and dimensions of the filling tubes 64, medication tube 67, and administration tube 60 can vary depending on the application.

The container 10 and the peelable seals 32 and 42 can be constructed from films able to make peal seal layers in accordance with embodiments of the present disclosure. The peelable seal layer films can allow both peelable and permanent seals to be created. Thus, the permanent side seals 14, 16, 18, and 20 as well as the peelable seals 32, 42, and 52 can be created from the same layer of film.

In the illustrated embodiment, any portion of the container 10 can be made from one or more suitable polymer materials. Suitable polymer materials include polyethylene homopolymers, ethylene α-olefin copolymers, polyethylene copolymers, polypropylene homopolymers, polypropylene copolymers, styrene and hydrocarbon random copolymers, styrene and hydrocarbon block copolymers, or combinations thereof. More examples of suitable polymer materials are described below.

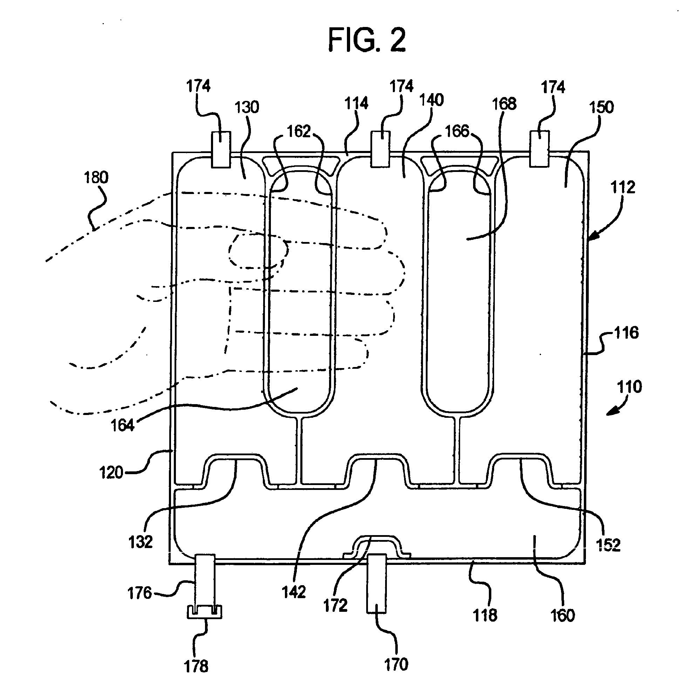

In another embodiment shown in FIG. 2, the present disclosure provides a multi-chambered container 110 including a body 112 made of at least two plies of a flexible polymer film defining a first chamber 130, a second chamber 140, a third chamber 150, and a fourth chamber 160 formed between the plies. The container 110 may be made from, for example, two or more plies of the film that are sealed about the periphery of the container 110 at edges 114, 116, 118 and 120.

A first peelable seal 132 separates the first chamber 130 and the fourth chamber 160. The first peelable seal 132 is independently openable by selective application of external pressure to the first chamber 130. A second peelable seal 142 separates the second chamber 140 and the fourth chamber 160. The second peelable seal 142 is independently openable by selective application of external pressure to the second chamber 140. A third peelable seal 152 separates the third chamber 150 and the fourth chamber 160. The third peelable seal 152 is independently openable by selective application of external pressure to the third chamber 150.

The first chamber 130 is separated from the second chamber 140 by a permanent seal 162 defining a transverse opening 164 through the plies. The second chamber 140 is separated from the third chamber 150 by a permanent seal 166 defining a transverse opening 168 through the plies. The first chamber 130, second chamber 140, and the third chamber 150 can be dimensioned to facilitate one-handed gripping of the chamber to apply the external pressure.

In an embodiment, the transverse openings 164 and 168 have a width (side to side) greater than 5 mm. The transverse openings 164 and 168 can also have a larger width such as, for example, a width greater than 6 mm, greater than 7 mm, greater than 8 mm, greater than 9 mm, greater than 10 mm, and the like. In another embodiment, the openings 164 and 168 have a length (top to bottom) greater than 5 cm. The transverse openings 164 and 168 can also have a larger length such as, for example, a width greater than 6 cm, greater than 7 cm, greater than 8 cm, greater than 9 cm, greater than 10 cm, greater than 11 cm, greater than 12 cm, greater than 13 cm, greater than 14 cm, greater than 15 cm, greater than 16 cm, greater than 17 cm, greater than 18 cm, greater than 19 cm, greater than 20 cm, and the like. For example, the transverse openings 164 and 168 can be dimensioned to admit a human hand 70 to apply the external pressure.

In an embodiment, the fourth chamber 160 is sized to facilitate mixing of the contents of any combination of the first, second, and third chambers 130, 140, and 150. The container 110 can further include a fluid outlet port 170 in fluid communication with the fourth chamber 160. A peelable safety seal 172 can separate the outlet port 170 from the fourth chamber 160. The safety seal 172 can have a seal strength selected to impede opening of the safety seal 172 unless at least one of the first, second, and third peelable seals 132, 142, and 152 have first been opened.

The container 110 can also include one or more tubes 174 in fluid communication with at least one of the first chamber 130, the second chamber 140, and the third chamber 150. Tubes 174 may function primarily as fill ports for the chambers 130, 140, and 150 and could be permanently sealed after filling, but may also be provided with a resealable closure to allow components to be added to or removed from the corresponding chambers. Similarly, the container 110 can also include one or more tubes 176 in fluid communication with chamber 160. Tube 176 can be equipped with a septum 178 or similar resealable closure so that medications or other additives may be added to chamber 160 without compromising the integrity of the container.

It should be appreciated that multi-chambered containers having more than 3 storage chambers and/or more than 1 mixing chamber can be made in accordance with alternative embodiments of the present disclosure. For example, the multi-chambered containers can have 4, 5, 6, 7, 8, or more storage chambers in combination with one or more mixing chambers.

In yet another embodiment, the present disclosure provides a method of administering a product such as, for example, a medical or a pharmaceutical solution. The method comprises providing a container including a first storage chamber having a first component, a second storage chamber having a second component, and a third mixing chamber. The container is constructed and arranged according to various embodiments of the present disclosure.

The method further comprises compressing at least one of the first storage chamber and the second storage chamber to allow a specified amount of a component from the corresponding compressed chamber to flow through into the mixing chamber. For example, continuous squeezing at a certain pressure can open the individual peelable seals allowing the desired amount of the contents of the corresponding chamber to flow into the mixing chamber. The squeezing can be performed by a user's hand squeezing the specific chamber.

The method can further comprise compressing the remaining chamber to allow a specified amount of component from the remaining compressed chamber to flow through into the mixing chamber to form a mixed component. The components from the first and second chambers can be mixed in the third chamber and administered through a fluid outlet port in fluid communication with the third chamber to a patient. The peelable seals can be reclosed to prevent the mixed solution from returning to the storage chambers and/or for storing any remaining component in the storage chambers.

The containers in alternative embodiments of the present disclosure can be fabricated using standard sealing techniques. For example, the container can be typically formed by placing one or more polymeric film plies forming the first sidewall and second sidewall of the container body in registration by their peripheral portions and sealing their periphery to form a liquid tight pouch having an outer permanent seal. The polymeric film plies can be sealed, for example, by heat sealing, radio frequency sealing, thermal transfer welding, adhesive sealing, solvent bonding, and ultrasonic or laser welding.

The peelable seals can be formed prior to, during, or after forming the permanent seal and can be made using appropriate sealing techniques such as, for example, heat conduction. The welding die for the peelable seals may have different temperatures and shapes along its length and/or edges to achieve the desired peelable seal configurations.

Blown extrusion is another method that may be used to make the pouch. Blown extrusion is a process that provides a moving tube of extrudate exiting an extrusion die. Air under pressure inflates the tube. Longitudinal ends of the tube are sealed to form the pouch. Blown extrusion only requires seals along two peripheral surfaces, where the single or multiple sheet registration method requires seals along three or four peripheral surfaces to form the pouch.

### The Sidewall Materials and Layer Structures

The containers 10 and 110 can be made principally of flexible polymeric materials, although the container could include non-polymeric materials such as metal foils without departing from the disclosure. Numerous polymeric films have been developed for use in containers. Suitable films may be of a monolayer structure or a multiple layer structure. The monolayer structure can be made from a single polymer, or from a polymer blend. The multiple layer structures can include layers such as a solution contact layer, a scratch resistant layer, a barrier layer for preventing permeation of gas (such as carbon dioxide, oxygen or water vapor), tie layers, or other layers. It is also contemplated to use more than one web of film for one or both sidewalls. Selection of the appropriate film depends on the solution or solutions to be contained within the container. Appropriate polymeric materials are generally selected from homopolymers and copolymers of polyolefins, polyamides, polyesters, polybutadiene, styrene and hydrocarbon copolymers, polyimides, polyester-polyethers, polyamide-polyethers to name a few.

The seal layer for a multi-chambered container should display bi-modal behavior. What is meant by bi-modal behavior is that the material is capable of forming a permanent seal under one set of sealing or manufacturing conditions and a peelable seal at a second set of sealing or manufacturing conditions. The seal layer can be a homophase polymer, or a matrix-phase polymer system. Suitable homophase polymers include polyolefins and more preferably polypropylene and most preferably a propylene and ethylene copolymer as described in EP 0875231.

It is also possible to have a seal layer having container walls of differing materials that are not compatible with one another. U.S. patent application Ser. No. 10/351,004, discloses that containers made from such incompatible material, in some instances, may not readily form permanent seals. This problem can be overcome by wrapping a section of one sidewall over an outside surface of the opposite sidewall and joined thereto. This method of sealing is disclosed in U.S. Pat. No. 6,024,220.

Suitable matrix-phase polymer systems will have at least two components. The two components can be blended together or can be produced in a two-stage reactor process. Typically, the two components will have different melting point or glass transition temperatures. In the case where one of the components is amorphous, its glass transition temperature will be lower than the melting point of the other components. Examples of suitable matrix-phase polymer system includes a component of a homopolymer or copolymer of a polyolefin and a second component of a styrene and hydrocarbon copolymer. Another suitable matrix-phase system includes blends of polyolefins such as polypropylene with polyethylene, or polypropylene with a high isotactic index (crystalline) with polypropylene with a lower isotactic index (amorphous), or a polypropylene homopolymer with a propylene and α-olefin copolymer.

Suitable polyolefins include homopolymers and copolymers obtained by polymerizing alpha-olefins containing from 2 to 20 carbon atoms, and more preferably from 2 to 10 carbons. Therefore, suitable polyolefins include polymers and copolymers of propylene, ethylene, butene-1, pentene-1,4-methyl-1-pentene, hexene-1, heptene-1, octene-1, nonene-1 and decene-1. Most preferably the polyolefin is a homopolymer or copolymer of propylene or a homopolymer or copolymer of polyethylene.

Suitable homopolymers of polypropylene can have a stereochemistry of amorphous, isotactic, syndiotactic, atactic, hemiisotactic or stereoblock. In one preferred form of the disclosure, the homopolymer of polypropylene is obtained using a single site catalyst.

Suitable copolymers of propylene are obtained by polymerizing a propylene monomer with an α-olefin having from 2 to 20 carbons. In a more preferred form of the disclosure, the propylene is copolymerized with ethylene in an amount by weight from about 1% to about 20%, more preferably from about 1% to about 10% and most preferably from 2% to about 5% by weight of the copolymer. The propylene and ethylene copolymers may be random or block copolymers. The propylene copolymer may also be obtained using a single site catalyst.

It is also possible to use a blend of polypropylene and α-olefin copolymers wherein the propylene copolymers can vary by the number of carbons in the α-olefin. For example, the present disclosure contemplates blends of propylene and α-olefin copolymers wherein one copolymer has a 2 carbon α-olefin and another copolymer has a 4 carbon α-olefin. It is also possible to use any combination of α-olefins from 2 to 20 carbons and most preferably from 2 to 8 carbons. Accordingly, the present disclosure contemplates blends of propylene and α-olefin copolymers wherein a first and second α-olefins have the following combination of carbon numbers: 2 and 6, 2 and 8, 4 and 6, 4 and 8. It is also contemplated using more than 2 polypropylene and α-olefin copolymers in the blend. Suitable polymers can be obtained using a catalloy procedure. Suitable homopolymers of ethylene include those having a density of greater than 0.915 g/cc and includes low density polyethylene (LDPE), medium density polyethylene (MDPE) and high density polyethylene (HDPE).

Suitable copolymers of ethylene are obtained by polymerizing ethylene monomers with an α-olefin having from 3 to 20 carbons, more preferably 3-10 carbons and most preferably from 4 to 8 carbons. It is also desirable for the copolymers of ethylene to have a density as measured by ASTM D-792 of less than about 0.915 g/cc and more preferably less than about 0.910 g/cc and even more preferably less than about 0.900 g/cc. Such polymers are oftentimes referred to as VLDPE (very low density polyethylene) or ULDPE (ultra low density polyethylene). Preferably the ethylene α-olefin copolymers are produced using a single site catalyst and even more preferably a metallocene catalyst systems. Single site catalysts are believed to have a single, sterically and electronically equivalent catalyst position as opposed to the Ziegler-Natta type catalysts which are known to have a mixture of catalysts sites. Such single-site catalyzed ethylene α-olefins are sold by Dow under the trade name AFFINITY, DuPont Dow under the trademark ENGAGE®, Eastman Kodak under the trade name MXSTEN, and by Exxon under the trade name EXACT. These copolymers shall sometimes be referred to herein as m-ULDPE.

Suitable copolymers of ethylene also include ethylene and lower alkyl acrylate copolymers, ethylene and lower alkyl substituted alkyl acrylate copolymers and ethylene vinyl acetate copolymers having a vinyl acetate content of from about 8% to about 40% by weight of the copolymer. The term "lower alkyl acrylates" refers to comonomers having the formula set forth in Diagram 1:

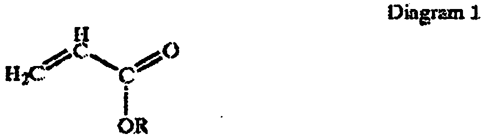

The R group refers to alkyls having from 1 to 17 carbons. Thus, the term "lower alkyl acrylates" includes but is not limited to methyl acrylate, ethyl acrylate, butyl acrylate and the like.

The term "alkyl substituted alkyl acrylates" refers to comonomers having the formula set forth in Diagram 2:

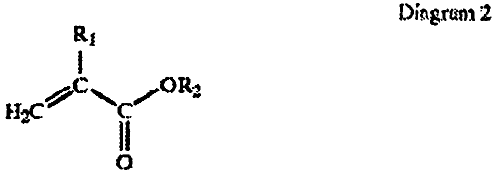

R₁ and R₂ are alkyls having 1-17 carbons and can have the same number of carbons or have a different number of carbons. Thus, the term "alkyl substituted alkyl acrylates" includes but is not limited to methyl methacrylate, ethyl methacrylate, methyl ethacrylate, ethyl ethacrylate, butyl methacrylate, butyl ethacrylate and the like.

Suitable polybutadienes include the 1,2- and 1,4-addition products of 1,3-butadiene (these shall collectively be referred to as polybutadienes). In a more preferred form of the disclosure, the polymer is a 1,2-addition product of 1,3 butadiene (these shall be referred to as 1,2 polybutadienes). In an even more preferred form of the disclosure, the polymer of interest is a syndiotactic 1,2-polybutadiene and even more preferably a low crystallinity, syndiotactic 1,2 polybutadiene. In a preferred form of the disclosure, the low crystallinity, syndiotactic 1,2 polybutadiene will have a crystallinity less than 50%, more preferably less than about 45%, even more preferably less than about 40%, even more preferably the crystallinity will be from about 13% to about 40%, and most preferably from about 15% to about 30%. In a preferred form of the disclosure, the low crystallinity, syndiotactic 1,2 polybutadiene will have a melting point temperature measured in accordance with ASTM D 3418 from about 70 °C to about 120 °C. Suitable resins include those sold by JSR (Japan Synthetic Rubber) under the grade designations: JSR RB 810, JSR RB 820, and JSR RB 830.

Suitable polyesters include polycondensation products of di-or polycarboxylic acids and di or poly hydroxy alcohols or alkylene oxides. In a preferred form of the disclosure, the polyester is a polyester ether. Suitable polyester ethers are obtained from reacting 1,4 cyclohexane dimethanol, 1,4 cyclohexane dicarboxylic acid and polytetramethylene glycol ether and shall be referred to generally as PCCE. Suitable PCCE's are sold by Eastman under the trade name ECDEL. Suitable polyesters further include polyester elastomers which are block copolymers of a hard crystalline segment of polybutylene terephthalate and a second segment of a soft (amorphous) polyether glycols. Such polyester elastomers are sold by DuPont Chemical Company under the trade name HYTREL®.

Suitable polyamides include those that result from a ring-opening reaction of lactams having from 4-12 carbons. This group of polyamides therefore includes nylon 6, nylon 10 and nylon 12. Acceptable polyamides also include aliphatic polyamides resulting from the condensation reaction of di-amines having a carbon number within a range of 2-13, aliphatic polyamides resulting from a condensation reaction of di-acids having a carbon number within a range of 2-13, polyamides resulting from the condensation reaction of dimer fatty acids, and amide containing copolymers. Thus, suitable aliphatic polyamides include, for example, nylon 6,6, nylon 6,10 and dimer fatty acid polyamides.

Suitable styrene and hydrocarbon copolymers include styrene and the various substituted styrenes including alkyl substituted styrene and halogen substituted styrene. The alkyl group can contain from 1 to about 6 carbon atoms. Specific examples of substituted styrenes include alpha-methylstyrene, beta-methylstyrene, vinyltoluene, 3-methylstyrene, 4-methylstyrene, 4-isopropylstyrene, 2,4-dimethylstyrene, o-chlorostyrene, p-chlorostyrene, o-bromostyrene, 2-chloro-4-methylstyrene, etc. Styrene is the most preferred.

The hydrocarbon portion of the styrene and hydrocarbon copolymer includes conjugated dienes. Conjugated dienes which may be utilized are those containing from 4 to about 10 carbon atoms and more generally, from 4 to 6 carbon atoms. Examples include 1,3-butadiene, 2-methyl-1,3-butadiene (isoprene), 2,3-dimethyl-1,3-butadiene, chloroprene, 1,3-pentadiene, 1,3-hexadiene, etc. Mixtures of these conjugated dienes also may be used such as mixtures of butadiene and isoprene. The preferred conjugated dienes are isoprene and 1,3-butadiene.

The styrene and hydrocarbon copolymers can be block copolymers including di-block, tri-block, multi-block, star block and mixtures of the same. Specific examples of di-block copolymers include styrene-butadiene, styrene-isoprene, and the hydrogenated derivatives thereof. Examples of tri-block polymers include styrene-butadiene-styrene, styrene-isoprene-styrene, alpha-methylstyrene-butadiene-alpha-methylstyrene, and alpha-methylstyrene-isoprene-alpha-methylstyrene and hydrogenated derivatives thereof.

The selective hydrogenation of the above block copolymers may be carried out by a variety of well known processes including hydrogenation in the presence of such catalysts as Raney nickel, noble metals such as platinum, palladium, etc., and soluble transition metal catalysts. Suitable hydrogenation processes which can be used are those wherein the diene-containing polymer or copolymer is dissolved in an inert hydrocarbon diluent such as cyclohexane and hydrogenated by reaction with hydrogen in the presence of a soluble hydrogenation catalyst. Such procedures are described in U.S. Pat. Nos. 3,113,986 and 4,226,952.

Particularly useful hydrogenated block copolymers are the hydrogenated block copolymers of styrene-isoprene-styrene, such as a styrene-(ethylene/propylene)-styrene block polymer. When a polystyrene-polybutadiene-polystyrene block copolymer is hydrogenated, the resulting product resembles a regular copolymer block of ethylene and 1-butene (EB). As noted above, when the conjugated diene employed is isoprene, the resulting hydrogenated product resembles a regular copolymer block of ethylene and propylene (EP). One example of a commercially available selectively hydrogenated copolymer is KRATON® G-1652 which is a hydrogenated SBS tri-block having 30% styrene end blocks and a mid-block equivalent is a copolymer of ethylene and 1-butene (EB). This hydrogenated block copolymer is often referred to as SEBS. Other suitable SEBS or SIS copolymers are sold by Kuraray under the tradename SEPTON® and HYBRAR®. It may also be desirable to use graft modified styrene and hydrocarbon block copolymers by grafting an alpha,beta-unsaturated monocarboxylic or dicarboxylic acid reagent onto the selectively hydrogenated block copolymers described above.

The block copolymers of the conjugated diene and the vinyl aromatic compound are grafted with an alpha, beta-unsaturated monocarboxylic or dicarboxylic acid reagent. The carboxylic acid reagents include carboxylic acids per se and their functional derivatives such as anhydrides, imides, metal salts, esters, etc., which are capable of being grafted onto the selectively hydrogenated block copolymer. The grafted polymer will usually contain from about 0.1 to about 20%, and preferably from about 0.1 to about 10% by weight based on the total weight of the block copolymer and the carboxylic acid reagent of the grafted carboxylic acid. Specific examples of useful monobasic carboxylic acids include acrylic acid, methacrylic acid, cinnamic acid, crotonic acid, acrylic anhydride, sodium acrylate, calcium acrylate and magnesium acrylate, etc. Examples of dicarboxylic acids and useful derivatives thereof include maleic acid, maleic anhydride, fumaric acid, mesaconic acid, itaconic acid, citraconic acid, itaconic anhydride, citraconic anhydride, monomethyl maleate, monosodium maleate, etc. The styrene and hydrocarbon block copolymer can be modified with an oil such as the oil modified SEBS sold by the Shell Chemical Company under the product designation KRATON G2705.

The films used in the containers of the present disclosure can be a multiple layer film having a seal layer, an intermediate layer, and an external layer. Tie layers may be employed to attach the seal layer to the intermediate layer and to attach the intermediate layer to the external layer. In a preferred form of the disclosure the seal layer is a blend of polypropylene, an ethylene α-olefin copolymer and a styrene and hydrocarbon copolymer. In a more preferred form of the disclosure, the polypropylene is a polypropylene ethylene copolymer, the ethylene α-olefin copolymer is a LLDPE having a density of less than 0.915 g/cc and the styrene and hydrocarbon copolymer is a block copolymer and preferably a tri-block copolymer of styrene-ethylene-butylene-styrene or a blend of an SEBS tri-block with an SEBS di-block as a minor component. The relative proportions of the components are preferably from about 55% to 75% of the PP by weight, from 5% to 20% by weight of the LLDPE, and from 10% to 20% by weight of the SEBS. The ternary blend of the seal layer is capable of forming a permanent seal and a peelable seal at a temperature of from about 123 to 128°C. A permanent seal is achieved at sealing temperatures above 145 °C.

The intermediate layer may be selected from any of the polyamides set forth herein and most preferably is a blend of from about 85 to 98% polyamide 6 and from 2 to 15% polyamide 6I,6T. The external layer can be selected from polypropylene polymer. For example, the external layer can be a propylene ethylene copolymer with an ethylene content of less than 6% by weight of the copolymer.

The details of the films are more fully set out in U.S. patent application Ser. No. 09/439,826, filed Nov. 12, 1999.

Another suitable film can contain three layers: an external layer, intermediate layer, and seal layer. The external layer can be a reactor made polypropylene composition having a first component and a second component. The first component can be a polypropylene homopolymer and can be present in an amount by weight of the composition of 40%. The second component can be an ethylene-propylene rubber (ethylene 20% and propylene 80%) and can be present in an amount by weight of the composition of 60%. Suitable products for the external layer are sold by Mitsubishi Chemical Company under the trade name Zelas 7023. Zelas 7023 is the subject of U.S. Patent Application Publication No. 2001/0034416 A1.

The intermediate layer can be a polymer blend of Zelas 7023 70% by weight and 30% by weight of a random copolymer of styrene and butadiene that has been hydrogenated. Suitable random copolymers of styrene and butadiene are sold by JSR under the trade name Dynaron 2320 P.

The external layer can be a polymer blend of 60% by weight Zelas 7023 and 40% by weight of a random copolymer of propylene and ethylene such as the copolymer sold under the trade name Novatec EG 7C. The films can display bi-modal behavior with peelable seals being formed at sealing temperatures of about 125 °C and permanent seals are obtained at about 145°C.

Other suitable films for this application include those disclosed in U.S. Pat. Nos. 5,849,843; 5,998,019; 6,083,587; 6,297,046; 5,139,831; 5,577,369; and U.S. Application No. 2003/0077466 A1.

### EXAMPLES

The multi-chambered containers can contain the following components in the storage and mixing chambers in alternative embodiments:
- The mixing/holding chamber contains a base peritoneal dialysis solution. A glucose concentrate is in one of the storage chambers. Another glucose concentrate is in a second storage chamber. An amino acid concentrate is in a third storage chamber. A polyglucose concentrate is in a fourth storage chamber. This would allow a patient or care giver to selectively mix one or more concentrates into a ready-to-use improved solution (over the base solution initially contained in the holding/mixing chamber). The different glucose concentrates can provide an iso-osmolar solution or a low osmolarity solution or a high osmolar solution by adding none, one, or two glucose concentrate solution(s) into the holding/mixing chamber.
- The mixing/holding chamber contains a base peritoneal dialysis solution. One of the storage chambers contains a bicarbonate concentrate. A second storage chamber contains a glucose or polyglucose concentrate. Mixing the contents of the two storage chambers into the holding/mixing chamber would provide the patient/care giver with a ready-to-use bicarbonate buffered, polyglucose-based solution for a long dwell.
- The mixing/holding chamber contains a base peritoneal dialysis solution. One of the storage chambers contains a bicarbonate concentrate. A second storage chamber contains an amino acid concentrate. Mixing the contents of the two storage chambers into the holding/mixing chamber would provide the patient/care giver with a ready-to-use bicarbonate buffered nutritional amino acid based solution for infusion in conjunction with a meal.
- The mixing/holding chamber is initially empty. One of the storage chambers contains a glucose based solution. A second storage chamber contains and amino acid based solution. A third storage chamber contains a lipid based emulsion. A fourth chamber contains a trace element based solution. Mixing the contents of two or more storage chambers would give respectively nutritional solutions similar to the two-in-one or three-in-one solutions contained in conventional two chambered or three chambered bags that could be supplemented with trace elements if necessary. The trace elements can be contained in one of the storage chambers as described above.

It should be understood that various changes and modifications to the presently preferred embodiments described herein will be apparent to those skilled in the art.

## Claims

1. A container (10, 110) comprising:
a first chamber (30, 130), a second chamber (40, 140), and a third chamber (50, 160);
a first peelable seal (32, 132) separating the first and third chambers, the first peelable seal being independently openable by selective application of external pressure to the first chamber; and
a second peelable seal (42, 142) separating the second and third chambers, the second peelable seal being independently openable by selective application of external pressure to the second chamber, wherein the first chamber is separated from the second chamber by a permanent seal (52, 162) defining a transverse opening (54, 164),
wherein the transverse opening is dimensioned to admit a human hand to apply the external pressure and wherein at least one of the first chamber and second chamber is dimensioned to facilitate one-handed gripping of the individual chamber to apply the external pressure.

2. The container of Claim 1, wherein the third chamber (50) contains a parenterally administrable base solution.

3. The container of Claim 1, wherein the third chamber (50) is empty and is sized to contain the entire contents of the first chamber (30) and the second chamber (40).

4. The container of any one of the preceding claims, wherein the container (10) is made from a film comprising at least one material selected from the group consisting of polyolefins, polyamides, polyesters, polybutadiene, styrene and hydrocarbon copolymers, polyimides, polyester-polyethers, polyamide-polyethers, and combinations thereof.

5. The container of Claim 4, wherein the film comprises at least one material selected from the group consisting of polyethylene homopolymers, ethylene α-olefin copolymers, polyethylene copolymers, polypropylene homopolymers, polypropylene copolymers, styrene and hydrocarbon random copolymers, styrene and hydrocarbon block copolymers, and combinations thereof.

6. The container of any one of the preceding claims, wherein the first peelable seal (32) and the second peelable seal (42) are activated by a force ranging from about 3 N/15 mm to about 30 N/15 mm.

7. The container of any one of the preceding claims, wherein the first peelable seal (32) has a first activating force and the second peelable seal (42) has a second activating force, the second peelable seal activating force being greater than the first peelable seal activating force.

8. The container of Claim 7, wherein the difference between the first peelable seal activating force and the second peelable seal activating force is greater than about 1 N/15 mm and less than about 5 N/15 mm.

9. The container of any one of the preceding claims further comprising a fluid outlet port (60) in fluid communication with the third chamber (50).

10. The container of Claim 9 further comprising a peelable safety seal (62) separating the outlet port (60) from the third chamber (50), wherein the safety seal has a seal strength selected to impede opening of the safety seal unless at least one of the first and second peelable seals (32, 42) have first been opened.

11. The container of any one of the preceding claims further comprising a tube (64) in fluid communication with at least one of the first chamber (30) and the second chamber (40).

12. The container of any one of the preceding claims, wherein the first chamber (30), second chamber (40) and third chamber (50) are formed between at least two plies of a flexible polymer film and wherein the transverse opening (54) is defined through the plies.

13. The container of claim 12, further comprising:
a fourth chamber (150) formed between the plies; and
a third peelable seal (152) separating the third and fourth chambers (160, 150), the third peelable seal being independently openable by selective application of external pressure to the fourth chamber;
wherein the permanent seal (162) separating the first chamber (130) from the second chamber (140) is a first permanent seal and wherein the second chamber (140) is separated from the fourth chamber (150) by a second permanent seal (166) that defines a second transverse opening (168) through the plies, both transverse openings being dimensioned to admit a human hand to apply the external pressure, and
wherein at least one of the first chamber, second chamber, and the fourth chamber is dimensioned to facilitate one-handed gripping of the chamber to apply the external pressure.

## Patentansprüche

1. Behälter (10, 110), umfassend:
eine erste Kammer (30, 130), eine zweite Kammer (40, 140) und eine dritte Kammer (50, 160),
ein erstes abziehbares Siegel (32, 132), welches die ersten und dritten Kammern trennt, wobei das erste abziehbare Siegel durch selektives Anwenden von äußerem Druck auf die erste Kammer unabhängig geöffnet werden kann, und
ein zweites abziehbares Siegel (42, 142), welches die zweiten und dritten Kammern trennt, wobei das zweite abziehbare Siegel durch selektives Anwenden von äußerem Druck auf die zweite Kammer unabhängig geöffnet werden kann, wobei die erste Kammer von der zweiten Kammer durch ein permanentes Siegel (52, 162), welches eine transversale Öffnung (54, 164) definiert, getrennt ist,
wobei die transversale Öffnung dimensioniert ist, um einer menschlichen Hand zu gestatten, den äußeren Druck anzuwenden, und wobei mindestens eine der ersten Kammer und zweiten Kammer dimensioniert ist, um einhändiges Ergreifen der einzelnen Kammer, um den äußeren Druck anzuwenden, zu erleichtern.

2. Behälter nach Anspruch 1, wobei die dritte Kammer (50) eine parenteral verabreichbare Basislösung enthält.

3. Behälter nach Anspruch 1, wobei die dritte Kammer (50) leer ist und dafür ausgelegt ist, um den gesamten Inhalt der ersten Kammer (30) und der zweiten Kammer (40) zu enthalten.

4. Behälter nach einem der vorhergehenden Ansprüche, wobei der Behälter (10) aus einer Folie, umfassend mindestens ein Material, ausgewählt aus der Gruppe, bestehend aus Polyolefinen, Polyamiden, Polyestern, Polybutadien-, Styrol- und Kohlenwasserstoff-Copolymeren, Polyimiden, Polyester-Polyethern, Polyamid-Polyethern und Kombinationen davon, hergestellt ist.

5. Behälter nach Anspruch 4, wobei die Folie mindestens ein Material, ausgewählt aus der Gruppe, bestehend aus Polyethylen-Homopolymeren, Ethylen-α-Olefin-Copolymeren, Polyethylen-Copolymeren, Polypropylen-Homopolymeren, Polypropylen-Copolymeren, Styrol- und Kohlenwasserstoff-Zufallscopolymeren, Styrol- und Kohlenwasserstoff-Blockcopolymeren und Kombinationen davon, umfasst.

6. Behälter nach einem der vorhergehenden Ansprüche, wobei das erste abziehbare Siegel (32) und das zweite abziehbare Siegel (42) durch eine Kraft von etwa 3 N/15 mm bis etwa 30 N/15 mm aktiviert werden.

7. Behälter nach einem der vorhergehenden Ansprüche, wobei das erste abziehbare Siegel (32) eine erste Aktivierungskraft aufweist und das zweite abziehbare Siegel (42) eine zweite Aktivierungskraft aufweist, wobei die zweite Aktivierungskraft des abziehbaren Siegels größer als die erste Aktivierungskraft des abziehbaren Siegels ist.

8. Behälter nach Anspruch 7, wobei der Unterschied zwischen der ersten Aktivierungskraft des abziehbaren Siegels und der zweiten Aktivierungskraft des abziehbaren Siegels größer als etwa 1 N/15 mm und weniger als etwa 5 N/15 mm ist.

9. Behälter nach einem der vorhergehenden Ansprüche, weiter umfassend eine Flüssigkeitsaustrittsöffnung (60) in Flüssigkeits-Verbindung mit der dritten Kammer (50).

10. Behälter nach Anspruch 9, weiter umfassend ein abziehbares Sicherheitssiegel (62), welches die Austrittsöffnung (60) von der dritten Kammer (50) trennt, wobei das Sicherheitssiegel eine Siegelstärke aufweist, ausgewählt, um das Öffnen des Sicherheitssiegels zu verhindern, sofern nicht mindestens eines der ersten und zweiten abziehbaren Siegel (32, 42) zuvor geöffnet wurden.

11. Behälter nach einem der vorhergehenden Ansprüche, weiter umfassend ein Rohr (64) in Flüssigkeits-Verbindung mit mindestens einer der ersten Kammer (30) und der zweiten Kammer (40).

12. Behälter nach einem der vorhergehenden Ansprüche, wobei die erste Kammer (30), zweite Kammer (40) und dritte Kammer (50) zwischen mindestens zwei Lagen einer flexiblen Polymerfolie gebildet sind, und wobei die transversale Öffnung (54) durch die Lagen definiert ist.

13. Behälter nach Anspruch 12, weiter umfassend
eine vierte Kammer (150), gebildet zwischen den Lagen, und
ein drittes abziehbares Siegel (152), welches die dritten und vierten Kammern (160, 150) trennt, wobei das dritte abziehbare Siegel durch selektives Anwenden von äußerem Druck auf die vierte Kammer unabhängig geöffnet werden kann,
wobei das permanente Siegel (162), welches die erste Kammer (130) von der zweiten Kammer (140) trennt, ein erstes permanentes Siegel ist, und wobei die zweite Kammer (140) von der vierten Kammer (150) durch ein zweites permanentes Siegel (166), welches eine zweite transversale Öffnung (168) durch die Lagen definiert, getrennt ist, wobei die beiden transversalen Öffnungen dimensioniert sind, um einer menschlichen Hand das Anwenden des äußeren Drucks zu gestatten, und
wobei mindestens eine der ersten Kammer, zweiten Kammer und der vierten Kammer dimensioniert ist, um einhändiges Ergreifen der Kammer, um den äußeren Druck anzuwenden, zu erleichtern.

## Revendications

1. Conteneur (10, 110) comprenant :
un premier compartiment (30, 130), un deuxième compartiment (40, 140), et un troisième compartiment (50, 160) ;
un premier scellement pouvant être rompu (32, 132) séparant les premier et troisième compartiments, le premier scellement pouvant être rompu pouvant être ouvert de manière indépendante par application sélective d'une pression externe sur le premier compartiment ; et
un deuxième scellement pouvant être rompu (42, 142) séparant les deuxième et troisième compartiments, le deuxième scellement pouvant être rompu pouvant être ouvert de manière indépendante par application sélective d'une pression externe sur le deuxième compartiment, dans lequel le premier compartiment est séparé du deuxième compartiment par un scellement permanent (52, 162) définissant une ouverture transversale (54, 164),
dans lequel l'ouverture transversale est dimensionnée de manière à admettre une main humaine afin d'appliquer la pression externe et dans lequel au moins l'un du premier compartiment et du deuxième compartiment est dimensionné de manière à faciliter la saisie à une main du compartiment individuel afin d'appliquer la pression externe.

2. Conteneur selon la revendication 1, dans lequel le troisième compartiment (50) contient une solution de base pouvant être administrée de manière parentérale.

3. Conteneur selon la revendication 1, dans lequel le troisième compartiment (50) est vide et est dimensionné de manière à contenir la totalité du contenu du premier compartiment (30) et du deuxième compartiment (40).

4. Conteneur selon l'une quelconque des revendications précédentes, dans lequel le conteneur (10) est réalisé à partir d'un film comprenant au moins un matériau sélectionné à partir du groupe constitué par des polyoléfines, des polyamides, des polyesters, du polybutadiène, des co-polymères de styrène et hydrocarbure, des polyimides, des polyester-polyéthers, des polyamide-polyéthers, et des mélanges de ceux-ci.

5. Conteneur selon la revendication 4, dans lequel le film comprend au moins un matériau sélectionné à partir du groupe constitué par des homopolymères de polyéthylène, des copolymères d'éthylène α-oléfine, des copolymères de polyéthylène, des homopolymères de polypropylène, des copolymères de polypropylène, des copolymères aléatoires de styrène et hydrocarbure, des copolymères bloc de styrène et hydrocarbure, et des mélanges de ceux-ci.

6. Conteneur selon l'une quelconque des revendications précédentes, dans lequel le premier scellement pouvant être rompu (32) et le deuxième scellement pouvant être rompu (42) sont activés par un effort compris entre 3 N/15 mm environ et 3 0 N/15 mm environ.

7. Conteneur selon l'une quelconque des revendications précédentes, dans lequel le premier scellement pouvant être rompu (32) présente un premier effort d'activation et le deuxième scellement pouvant être rompu (42) présente un deuxième effort d'activation, l'effort d'activation du deuxième scellement pouvant être rompu étant supérieur à l'effort d'activation du premier scellement pouvant être rompu.

8. Conteneur selon la revendication 7, dans lequel la différence entre l'effort d'activation du premier scellement pouvant être rompu et l'effort d'activation du deuxième scellement pouvant être rompu est supérieure à 1 N/15 mm environ et inférieure à 5 N/15 mm environ.

9. Conteneur selon l'une quelconque des revendications précédentes, comprenant, en outre, un orifice de sortie de fluide (60) en communication fluidique avec le troisième compartiment (50).

10. Conteneur selon la revendication 9, comprenant, en outre, un scellement de sécurité pouvant être rompu (62) séparant l'orifice de sortie (60) du troisième compartiment (50), dans lequel le scellement de sécurité présente une résistance de scellement sélectionnée afin d'empêcher l'ouverture du scellement de sécurité sauf si au moins l'un des premier et deuxième scellements pouvant être rompus (32, 42) a d'abord été ouvert.

11. Conteneur selon l'une quelconque des revendications précédentes, comprenant, en outre, un tube (64) en communication fluidique avec au moins l'un du premier compartiment (30) et du deuxième compartiment (40).

12. Conteneur selon l'une quelconque des revendications précédentes, dans lequel le premier compartiment (30), le deuxième compartiment (40) et le troisième compartiment (50) sont formés entre au moins deux couches d'un film polymère flexible et dans lequel l'ouverture transversale (54) est définie à travers les couches.

13. Conteneur selon la revendication 12, comprenant, en outre :
un quatrième compartiment (150) formé entre les couches ; et
un troisième scellement pouvant être rompu (152) séparant les troisième et quatrième compartiments (160, 150), le troisième scellement pouvant être rompu pouvant être ouvert de manière indépendante par application sélective d'une pression externe sur le quatrième compartiment ;
dans lequel le scellement permanent (162) séparant le premier compartiment (130) du deuxième compartiment (140) est un premier scellement permanent et dans lequel le deuxième compartiment (140) est séparé du quatrième compartiment (150) par un deuxième scellement permanent (166) qui définit une deuxième ouverture transversale (168) à travers les couches, les deux ouvertures transversales étant dimensionnées de manière à admettre une main humaine afin d'appliquer la pression externe, et
dans lequel au moins l'un du premier compartiment, du deuxième compartiment, et du quatrième compartiment est dimensionné de manière à faciliter la saisie à une main du compartiment afin d'appliquer la pression externe.
